# EUROPÄISCHE PATENTSCHRIFT

(11) **EP 3 095 773 B1**
(45) Veröffentlichungstag und Bekanntmachung des Hinweises auf die Patenterteilung: **02.10.2019**
(21) Anmeldenummer: 15168597.1
(22) Anmeldetag: 21.05.2015
(51) Int. Cl.: C07C 29/132, C07C 35/17, C07C 35/18, C07C 407/00, C07C 409/14

(54) **HERSTELLUNG VON LIMONENDERIVATEN**
PREPARATION OF LIMONENE DERIVATIVES
FABRICATION DE DÉRIVÉS DE LIMONÈNE

(43) Veröffentlichungstag der Anmeldung: 23.11.2016
(73) Patentinhaber: Symrise AG, 37603 Holzminden (DE)
(72) Erfinder: Dilk, Erich, 37603 Holzminden (DE); Geisel, Detlef, 37603 Holzminden (DE); Lambrecht, Stefan, 37619 Hehlen (DE)
(74) Vertreter: Global IP Europe Patentanwaltskanzlei

(56) Entgegenhaltungen:
- WO-A2-2009/033247
- US-A- 3 014 047
- GÜNTHER O. SCHENCK ET AL: "Zur chemischen und sterischen Selektivität der photosensibilisierten O2-Übertragung auf (+)-Limonen und (+)-Carvomenthen", JUSTUS LIEBIGS ANNALEN DER CHEMIE, Bd. 674, Nr. 1, 9. Juli 1964 (1964-07-09), Seiten 93-117, XP55224411, WEINHEIM; DE ISSN: 0075-4617, DOI: 10.1002/jlac.19646740111

## Beschreibung

### Gebiet der Erfindung

Die Erfindung betrifft die Herstellung von Duft-, Geschmacksstoffen bzw. deren Zwischenverbindungen basierend auf der Oxidation von Limonen.

### Stand der Technik

Limonen ist eine chemische Verbindung, die in der Natur vorkommt und der Gruppe der Terpene zugeordnet wird. Es ist allgemein bekannt, dass Limonen als Edukt für die Herstellung von bestimmten Duft-, Geschmacksstoffen oder auch deren Zwischenverbindungen dient.

Es besteht der Bedarf, die Verbindungen p-2,8-Menthadien-1-ol, p-1(7),8-Menthadien-2-ol und Carveol herzustellen. p-2,8-Menthadien-1-ol kommt unter anderem in bestimmten Kräutern und bestimmten Zitrusfrüchten vor. Es wird z.B. in Aromen und Parfüms eingesetzt und kann ebenfalls als Edukt zur Synthese weiterer Verbindungen, zum Beispiel des Arzneimittels Tetrahydrocannabidiol verwendet werden. (Quelle: Kraatz, Korte: Sauerstoffanaloge Delta8- und Delta9 - Tetrahydrocannabinol, Z. Naturforsch. 31b. 1382-1386 [1976]) p-2,8-Menthadien-1-ol, p-1(7), 8-Menthadien-2-ol und Carveol können entweder selbst als Duft- oder Geschmacksstoffe eingesetzt werden oder können als Zwischenprodukt für weitere Verbindungen dienen.

Bisher bekannte Verfahren zur Herstellung von p-2,8-Menthadien-1-ol, p-1(7), 8-Menthadien-2-ol und Carveol umfassen oft die photochemische Oxidation von Limonen in Gegenwart von Wasserstoffperoxid (H₂O₂). Das dabei intermediär gebildete Hydroperoxid-Gemisch wird durch Behandlung mit Natriumsulfit-Lösung in das entsprechende Alkoholgemisch überführt (z.B. G.O. Schenk et al, Liebigs Ann. Chemie, 674 (1964), 93-117).

Der Nachteil photochemischer Herstellverfahren besteht darin, dass diese in speziellen Photoreaktoren durchgeführt werden. Diese Spezialausrüstungen erfordern in Vergleich zu üblichen chemischen Reaktoren höhere Investitionen und sind daher auch weniger verbreitet. Der Betrieb von Photoreaktoren ist zudem aufwändig.

Bösing et. al. in J. Am. Chem. Soc. 1998, 120, 7252-7259 offenbaren die Herstellung eines speziellen Isomers des p-2,8-Menthadien-1-ols aus Limonen und H₂O₂ unter Verwendung von Mangan-(II)-substituierten Polyoxometallaten mit sehr großem Molgewicht als Katalysatoren. Die Reaktion dauert sehr viele Stunden und liefert neben Limonenepoxiden lediglich das 1S, 4R Isomer des p-2,8-Menthadien-1-ols. Bei der Verwendung dieses isolierten Isomers treten allerdings geruchliche und geschmackliche Veränderungen im Vergleich zu der oft verwendeten Isomerenmischung, die bei der Photooxidation entsteht, ein. Zudem sind die verwendeten Katalysatoren relativ teuer, was die Wirtschaftlichkeit verringert. p-1(7),8-Menthadien-2-ol und Carveol entstehen nicht bei diesem Verfahren. Zur Herstellung dieser beiden wichtigen Duftstoffe, Geschmacksstoffe bzw. Zwischenverbindungen müssten bei der Anwendung dieses Verfahrens zusätzliche Verfahren mit zusätzlichem Aufwand entwickelt, etabliert und eingesetzt werden.

Das Dokument D1 WO 2009/033247 A2 offenbart ein Verfahren der katalytischen Oxidation eines ätherischen Öls aus *laranja pêra* (*citrus sinensis*), welches 96 % Limonen enthält, mit 35 %iger H₂O₂-Lösung in Gegenwart eines heterogenen Katalysators der allgemeine Formel L/MₓN_{y} mit M = Zr, Al, Si, Ti; N = O; x und y = 2 oder 3, und L = Co, Ti, V, Cr, Mn, Fe, Cu, Mo, W, Re, und in Abwesenheit eines Lösungsmittels, bei einer Temperatur von 25°C bis 120°C. Die offenbarte Reaktionsdauer beträgt ca. 24 Stunden und setzt ca. 50 % des Limonens um.

Es ist bisher schwierig oder annähernd unmöglich, die Verhältnisse der gebildeten Reaktionsprodukte wesentlich zu beeinflussen. Dies wäre jedoch wünschenswert, um auf schwankende Absatzmengen der Reaktionsprodukte reagieren zu können.

### Aufgabe der Erfindung

Die Aufgabe der Erfindung bestand unter anderem darin, die eingangs geschilderten Nachteile des Standes der Technik zu verbessern. Es kann auch als Aufgabe angesehen werden, eine industriell nutzbare Herstellweise für p-2,8-Menthadien-1-ol, p-1(7), 8-Menthadien-2-ol und Carveol zur Verfügung zu stellen, die keine Photooxidation und die damit verbundene Spezialausrüstung verwendet. Dabei sollten die produzierten Verbindungen über vorteilhafte sensorische (geruchliche und geschmackliche) Eigenschaften verfügen, die Reaktion schnell, wirtschaftlich und im industriellen Maßstab ablaufen. Das Isomerenverhältnis aus *cis-* und *trans* p-2,8-Menthadien-1-ol sollte annähernd dem Verhältnis aus dem bekannten Photooxidationsverfahren entsprechen, um geruchliche und geschmackliche Abweichungen vom bisherigen Produkt zu vermeiden.

Als Aufgabe kann auch angesehen werden, dass p-2,8-Menthadien-1-ol, p-1(7), 8-Menthadien-2-ol und Carveol in wirtschaftlich verwertbaren Mengen mit Hilfe eines einzigen Verfahrens hergestellt werden. Zudem sollten die gebildeten Mengenverhältnisse zwischen p-2,8-Menthadien-1-ol, p-1(7), 8-Menthadien-2-ol und Carveol beeinflussbar sein, um beispielsweise bei schwankenden Verkaufsmengen bedarfsgerecht produzieren zu können, wobei eine hohe Ausbeute an den Stoffen p-2,8-Menthadien-1-ol, p-1(7), 8-Menthadien-2-ol und Carveol in Summe wünschenswert ist. Zudem sollte nicht umgesetztes Limonen einfach wiedergewonnen werden können.

### Detaillierte Beschreibung der Erfindung

Die komplexe Aufgabe wird gelöst durch das in den Ansprüchen beschriebene Verfahren. Es gliedert sich in 2 Stufen. Die erste Stufe umfasst die Umsetzung von Limonen (d/I-Limonen, d-Limonen oder I-Limonen) mit Wasserstoffperoxid in Gegenwart eines Katalysators ausgewählt aus der Gruppe von Natriummolybdat, Natriummolybdat-Dihydrat, Natriumwolframat, Natriumwolframat-Dihydrat und Lanthannitrat.

Es werden ebenfalls Katalysatoren die Atome oder/und Ionen von Molybdän, Wolfram, Scandium, Vanadium, Titan und Lanthan und besonders bevorzugt von Molybdän und Wolfram enthalten, offenbart. Diese sind jedoch nicht Gegenstand der vorliegenden Erfindung. Die Metalle können auch in Form von beispielsweise Oxokomplexen, Oxiden, Hydroxiden, Salzen, wie z.B. Nitraten, Carboxylaten, Carbonaten, Chloriden, Fluoriden, Sulfaten oder Tetrafluoroboraten, vorliegen.

Die Katalysatoren können insbesondere als Feststoff oder in gelöster Form eingesetzt werden.

Die Reaktion kann insbesondere in mindestens einem organischen Lösungsmittel stattfinden. Beispielsweise können die Lösungsmittel aus der Gruppe der Alkohole mit 1 bis 8 C-Atomen und Amide ausgewählt werden. Auch können die Lösungsmittel aus der Gruppe von Methanol, Ethanol, Propanol, i-Propanol, Ethylenglykol, Propylenglykol, N-Methylformamid, Dimethylformamid oder N-Methylpyrrolidon ausgewählt werden. Optional kann das mindestens eine organische Lösungsmittel bis zu 30 Gew.-% Wasser enthalten.

Die Reaktion läuft vorteilhaft bei einem pH-Wert größer als 7,5, bevorzugt größer 8 und weiter bevorzugt größer 9 und bei Temperaturen zwischen 25 und 90°C, bevorzugt zwischen 40 und 90°C ab. Weitere vorteilhafte Ergebnisse können bei einer Katalysatormenge von 1 bis 50 Mol-% bezogen auf Limonen oder / und 2-10 Moläquivalente Wasserstoffperoxid pro 1 Mol Limonen erzielt werden.

Als Produkte der ersten Stufe entstehen überwiegend die Hydroperoxide des Limonens, welche in einer zweiten Stufe mit einem Reduktionsmittel umgesetzt werden. Das bevorzugte Reduktionsmittel ist Natriumsulfit. Die Reduktion kann beispielsweise durch Einleitung der Reaktionsprodukte der ersten Stufe in eine wässrige Natriumsulfitlösung und anschließendem Rühren bei 25 bis 90 °C und einem pH-Wert größer als 7,5 erfolgen.

Im Anschluss wird die erhaltene Reaktionsmischung durch übliche Trennverfahren, wie etwa fraktionierte Destillation aufgetrennt. Aus der Reaktionsmischung lassen sich insbesondere p-2,8-Menthadien-1-ol, p-1(7), 8-Menthadien-2-ol und Carveol gewinnen.

Die Umsetzungen können sowohl im Batch- als auch Konti-Verfahren durchgeführt werden. Während der Umsetzungen kann ein Stickstoffstrom durch die Reaktionsapparatur geleitet werden.

Bei einem möglichen jedoch nicht limitierenden Beispiel einer batchweisen Umsetzung werden Limonen, Methanol und der Katalysator, gegebenfalls in Wasser gelöst, vorgelegt und bei der gewählten Reaktionstemperatur Wasserstoffperoxid-Lösung zudosiert. Nach dem Reaktionsende werden die gebildeten Hydroperoxide durch Eindosieren in eine Natriumsulfitlösung reduziert.

Bei einem möglichen jedoch nicht limitierenden Beispiel einer kontinuierlichen Durchführung werden die Einsatzstoffe in den unteren Bereich in eines Rohrreaktors eindosiert und die abreagierte Reaktionsmischung im oberen Bereich abgenommen bzw. in die Natriumsulfit-Lösung geleitet.

### Beispiele

### Beispiel 1

In einer 100 mL-Dreihalskolbenrührapparatur werden 2,72 g D-Limonen in 24,4 g Methanol vorgelegt. Hierzu werden 0,22 g Natriummolybdat-Dihydrat, gelöst in 1,86 g Wasser, zugegeben und mit 5%iger Natronlauge auf pH = 10 eingestellt. Es wird auf Rückflusstemperatur erhitzt und 4 g Wasserstoffperoxid 50%ig innerhalb von 30 Minuten zudosiert und weitere 5 Minuten nachreagieren lassen. Das Reaktionsgemisch wird anschließend bei 60°C in eine Lösung von 2,5 g Natriumsulfit in 7,2 g Wasser gegeben und zur vollständigen Peroxidzersetzung 3 Stunden nachgerührt. Es wird vom ausgefallenen Niederschlag abfiltriert und das Filtrat am Rotationsverdampfer eingeengt. Die Rohproduktzusammensetzung nach GC-Flächen-% ist in Tabelle 1 angegeben.

### Beispiel 2

In einer 100 ml-Dreihalskolbenrührapparatur werden 2,72 g D-Limonen in 11 g Methanol und 7 g Wasser vorgelegt. Die weitere Durchführung erfolgte analog Beispiel 1.

### Beispiel 3

Analog Beispiel 2, jedoch mit 0,3 g Natriumwolframat Dihydrat, gelöst in 1,86 g Wasser.

### Beispiel 4

Analog Beispiel 1, jedoch mit 0,4 g Lanthannitrat, gelöst in 1,86 g Wasser.

In Tabelle 1 ist erkennbar, dass Ausbeute der hergestellten Mengen der Verbindungen p-2,8-Menthadien-1-ol, p-1(7), 8-Menthadien-2-ol und Carveol in Summe sehr hoch ist. Außerdem lässt erkennen, dass bei den unterschiedlichen Herstellungsvarianten das Verhältnis der Stoffe untereinander variiert.

### Beispiel 5

In einer 1L-Doppelmantelrührapparatur werden 27,2 g D-Limonen in 244 g Methanol vorgelegt. Hierzu werden 4,4 g Natriummolybdat-Dihydrat, gelöst in 18,6 g Wasser, gegeben und mit 1,5 g 5%iger Natronlauge auf pH = 10 eingestellt. Es wird auf Rückflusstemperatur erhitzt und 40,8 g Wasserstoffperoxid 50%ig innerhalb von 30 Minuten zudosiert und weitere 5 Minuten nachreagieren lassen. Das Reaktionsgemisch wird anschließend bei 60°C in eine Lösung von 25 g Natriumsulfit in 72 g Wasser gegeben und zur vollständigen Peroxidzersetzung 3 Stunden bei 60°C nachgerührt. Es wird vom ausgefallenen Niederschlag abfiltriert und mit tert.-Butylmethylether nachgespült. Vom erhaltenen Filtrat werden Methanol und tert.-Butylmethylether abdestilliert. Mit dem verbleibenden 2-phasigen Rückstand wird eine Phasentrennung durchgeführt und die wässrige Phase 2 Mal mit je 20 g tert.-Butylmethylether extrahiert. Die vereinigten organischen Phasen werden mittels Rotationsverdampfer eingeengt und der verbleibende Rückstand im Vakuum destilliert. Es werden 13,1 g Destillat erhalten.

### Beispiel 6 (kontinuierliche Verfahrensweise)

In ein Doppelmantelreaktionsrohr mit einem Volumen von 150 ml werden 13,6 g D-Limonen, 61 g Methanol und 1,1 g Natriummolybdat-Dihydrat, gelöst in 2 g Wasser, gegeben und 0,75 g 5%ige Natronlauge zugefügt. Das Reaktionsgemisch wird auf 60°C erwärmt und innerhalb von 30 Minuten werden hierzu 20,4 g Wasserstoffperoxid 50%ig in den unteren Bereich des Reaktors dosiert. Anschließend werden innerhalb von 45 Minuten mittels Dosierpumpen parallel 61,2 g Wasserstoffperoxid 50%ig sowie eine gerührte Mischung bestehend aus 40,8 g D-Limonen, 183 g Methanol, 3,3 g Natriummolybdat-Dihydrat, 18 g Wasser und 2,3 g 5%ige Natronlauge in den Bodenbereich des Reaktors dosiert. Die resultierende Reaktionsmischung wird aus dem oberen Reaktorbereich kontinuierlich in eine Lösung aus 50 g Natriumsulfit und 144 g Wasser geleitet und bei 60°C für 3 Stunden gerührt. Die weitere Aufarbeitung erfolgt analog Beispiel 5. Es werden 29,1 g Destillat erhalten.

**Tabelle 1: Anteile in der Reaktionsmischung nach dem Reduktionsschritt**

| Beispiel | GC-Flächen-% | | | | |
|---|---|---|---|---|---|
| | D-Limonen | *cis*/*trans* p-2,8-Menthadien-1-ol | *cis*/*trans* p-1 (7),8-Menthadien-2-ol | *cis*/*trans* Carveol | Summe der Produkte |
| | | (Produkt A) | (Produkt B) | (Produkt C) | A, B und C |
| 1 | 5,8 | 35,6 | 36 | 14,7 | 86,3 |
| 2 | 2,9 | 31,9 | 39 | 19,3 | 90,2 |
| 3 | 14,6 | 11,9 | 27,5 | 25,8 | 65,2 |
| 4 | 15 | 18,5 | 36,3 | 24,9 | 79,7 |
| 5 | n.n. | 40,9% | 38,5% | 17,8% | 97,2 |
| 6 | 27,7% | 28,2% | 29% | 11,5% | 68,7 |

### Analyse des cis/trans-Gehaltes von p-2,8-Menthadien-1-ol

Der Gehalt der *cis* / *trans* Isomere wird gaschromatographisch bestimmt. Das Verhältnis aus *cis* p-2,8-Menthadien-1-ol zu *trans* p-2,8-Menthadien-1-ol beträgt etwa 1:3 und entspricht damit annähernd dem Verhältnis aus Schenk et. al, Liebigs Ann. Chemie, 674 (1964), 93-117. Die Verhältnisse von *cis* zu *trans* p-1(7), 8-Menthadien-2-ol sowie von *cis* zu *trans* Carveol entsprechen ebenfalls den in Schenk et al. angegebenen Verhältnissen. Die Isomerenverhältnisse sind somit wie beim Photooxidationsverfahren.

## Patentansprüche

1. Verfahren zur Oxidation von Limonen umfassend die Umsetzung von Limonen mit Wasserstoffperoxid in Gegenwart eines Katalysators ausgewählt aus der Gruppe von Natriummolybdat, Natriummolybdat-Dihydrat, Natriumwolframat, Natriumwolframat-Dihydrat und Lanthannitrat.

2. Verfahren nach einem der vorhergehenden Ansprüche, **dadurch gekennzeichnet, dass** die Reaktion in mindestens einem organischen Lösungsmittel stattfindet.

3. Verfahren nach einem der vorhergehenden Ansprüche, **dadurch gekennzeichnet, dass** der pH-Wert größer als 7,5, bevorzugt größer 8 und weiter bevorzugt größer 9 ist.

4. Verfahren nach einem der vorhergehenden Ansprüche, **dadurch gekennzeichnet, dass** die Temperatur von 25 bis 90°C beträgt.

5. Verfahren nach einem der vorhergehenden Ansprüche, **dadurch gekennzeichnet, dass** die verwendete Katalysatormenge 1 bis 50 Mol-% bezogen auf Limonen beträgt.

6. Verfahren nach einem der vorhergehenden Ansprüche, **dadurch gekennzeichnet, dass** 2-10 Moläquivalente Wasserstoffperoxid pro 1 Mol Limonen verwendet werden.

7. Verfahren nach Anspruch 1 umfassend das Umsetzen des Reaktionsproduktes nach den Ansprüchen 1 bis 6 mit einem Reduktionsmittel, **dadurch gekennzeichnet, dass** es bei 25 bis 90°C, bei einem pH-Wert größer als 7,5 durchgeführt wird.

8. Verfahren nach Anspruch 7 umfassend das Auftrennen des Reaktionsproduktes, bevorzugt durch Destillation.

## Claims

1. Process for the oxidation of limonene, which comprises reacting limonene with hydrogen peroxide in the presence of a catalyst selected from the group consisting of sodium molybdate, sodium molybdate dihydrate, sodium tungstate, sodium tungstate dihydrate and lanthanum nitrate.

2. Process according to one of the preceding claims, **characterized in that** the reaction takes place in at least one organic solvent.

3. Process according to one of the preceding claims, **characterized in that** the pH value is greater than 7.5, preferably greater than 8 and more preferably greater than 9.

4. Process according to one of the preceding claims, **characterized in that** the temperature is from 25 to 90°C.

5. Process according to one of the preceding claims, **characterized in that** the amount of catalyst used is 1 to 50 mol% in relation to limonene.

6. Process according to one of the preceding claims, **characterized in that** 2-10 molar equivalents of hydrogen peroxide per 1 mole limonene are used.

7. Process according to claim 1, which comprises reacting the reaction product according to claims 1 to 6 with a reducing agent, **characterized in that** this is carried out at 25 to 90°C, at a pH value greater than 7.5.

8. Process according to claim 7, which comprises separating the reaction product, preferably by distillation.

## Revendications

1. Procédé d'oxydation de limonène, comprenant la conversion de limonène avec du peroxyde d'hydrogène en présence d'un catalyseur sélectionné dans le groupe comprenant molybdate de sodium, molybdate de sodium dihydraté, tungstate de sodium, tungstate de sodium dihydraté et nitrate de lanthane.

2. Procédé selon l'une des revendications précédentes, **caractérisé en ce que** la réaction a lieu dans au moins un solvant organique.

3. Procédé selon l'une des revendications précédentes, **caractérisé en ce que** la valeur pH est supérieure à 7,5, préférentiellement supérieure à 8 et tout particulièrement supérieure à 9.

4. Procédé selon l'une des revendications précédentes, **caractérisé en ce que** la température est comprise entre 25 et 90 °C.

5. Procédé selon l'une des revendications précédentes, **caractérisé en ce que** la quantité de catalyseur utilisée est comprise entre 1 et 50 % mol par rapport au limonène.

6. Procédé selon l'une des revendications précédentes, **caractérisé en ce que** 2 à 10 équivalents molaires de peroxyde d'hydrogène par mole de limonène sont utilisés.

7. Procédé selon la revendication 1, comprenant la conversion du produit de réaction selon les revendications 1 à 6 avec un agent réducteur, **caractérisé en ce que** celle-ci est effectuée entre 25 et 90 °C, pour une valeur pH supérieure à 7,5.

8. Procédé selon la revendication 7, comprenant la séparation du produit de réaction, préférentiellement par distillation.
